# EUROPEAN PATENT APPLICATION

(11) **EP 4 234 714 A1**
(43) Date of publication of application: **30.08.2023**
(21) Application number: 22382168.7
(22) Date of filing: 28.02.2022
(51) Int. Cl.: C12Q 1/6837, C12Q 1/6886

(54) **MULTIPLEXED SYSTEM FOR THE DETECTION OF DISEASE-RELATED MICRORNAS IN BIOLOGICAL SAMPLES**

(71) Applicant: Fundación Imdea Nanociencia, 28049 Madrid (ES)
(72) Inventor: Castanheira Coutinho, Catarina, Madrid (ES); Castellanos Molina, Milagros, Madrid (ES); Somoza Calatrava, Álvaro, Madrid (ES)
(74) Representative: Pons

(57) **Abstract**

The invention refers to metal nanoparticles and an *in vitro* method for the multiplex detection of disease-related nucleic acids, preferably miRNAs, more preferably cancer-related miRNAs, in biological samples. The method is based on the use of a multiplexed colorimetric sensor that comprises metal nanoparticles (NPs), preferably gold NPs, functionalized with different oligonucleotide probes that fold into a hairpin structure. In the presence of the complementary target sequence, the hairpin unfolds, which leads to the aggregation of the structure that can be detected with the naked eye.

## Description

The present invention falls within the field of nanobiotechnology and methods for the multiplex detection of nucleic acids, preferably microRNAs, involved in diseases, preferably cancer, through the use of functionalized metal nanoparticles (NPs). In particular, it refers to metal NPs that are functionalized with different oligonucleotide probes able to detect the presence of multiple disease-related nucleic acid sequences in a sample at a time, as well as to methods involving the use of said NPs and kits comprising the same.

### BACKGROUND ART

According to the WHO, cancer is the second leading cause of death globally. The early and precise detection of the disease is critical for the success of treatments and the reduction of cancer-associated mortality. In this regard, microRNAs (or miRNAs), a family of non-coding RNAs targeting more than 50% of protein-coding genes, are dysregulated in various diseases, including cancer, and have been proposed as non-invasive biomarkers in liquid biopsies for cancer early diagnosis, stratification of patients or evaluation of the response to therapy. Moreover, in the case of cancer, their dysregulation is associated with early cancer stages, and their expression levels vary among cancer subtypes and/or different stages of the disease. These molecules have been found in various fluids such as plasma, serum, urine, saliva, seminal and ascites fluids, amniotic pleural effusions, or cerebrospinal fluid. However, despite their convenient use as non-invasive biomarkers, the detection of these nucleic acids presents some difficulties, and their general use in clinics is still limited, essentially due to their specific properties and very low concentrations. Conventional methods like RT-PCR, microarray analysis, Northern blotting, polyacrylamide gel electrophoresis, or the more recent microarray and RNA-Seq platforms offer high accuracy and sensitivity in nucleic acid detection. However, these methods are not suitable for routine diagnosis because, first, they are complex and highly trained personnel and expensive equipment are required, and, second, they are time-consuming. For these reasons, the development of new detection systems, particularly those aiming to be point-of-care (PoC) devices, which allow fast, simple and cost-effective detection, is necessary. In this regard, the use of nanomaterials, in particular, nanoparticles (NPs), has eased the development of this kind of sensing devices. NPs have unique and valuable properties for the design of biosensors, such as high surface-to-volume ratio, high surface energy, tunable optical properties, high stability and biocompatibility. These features have improved the applicability of sensor systems in PoC, in terms of simplicity, sensitivity, selectivity, cost-effectiveness and detection speed.

Among the variety of nanostructures used to detect nucleic acids, gold nanoparticles (AuNPs) have been employed extensively due to their excellent optical properties and ease of preparation and modification. In this regard, AuNPs modified with oligonucleotides that present a stable folded structure that unfolds only in the presence of a complementary target sequence have been prepared. The opening of the structure exposes a molecule able to produce a signal. For instance, a fluorescent dye at the end of the oligonucleotide can be used as a reporter molecule, constituting the commonly named molecular beacons. In this case, the oligonucleotides, once conjugated to AuNPs, can be used for intracellular detection of RNA. In this system, the unfolding of the structure, upon nucleic acid hybridization, leads to an increase in fluorescence. Other types of oligonucleotides and reporter molecules have been used, such as biotin, promoting streptavidin-mediated AuNP aggregation, or retention on nitrocellulose strips, once exposed.

In particular, a detection system had been created for the detection of single-point mutations in RNA extracts (A. Latorre, et al., 2014, Chem. Commun., 50, 3018-20) and it was adapted for the detection of RNAs. The oligonucleotides used in these sensors are related to the molecular beacons, which are also hairpin-structured oligonucleotides, containing a fluorophore in one end and a quencher in the other, and are used as fluorescent probes for nucleic acid detection (S. Tyagi, F. R. Kramer, 1996, Nat. Biotechnol, 14, 303-308). These molecules have been conjugated to AuNPs (where the nanoparticle functions both as quencher and delivery vehicle) and used for intracellular detection of mRNA (C. Posch, et al., 2015, Biomed. Microdevices, 17, 15). Other types of oligonucleotides and reporter molecules (instead of fluorophores) have been conjugated to AuNPs, such as biotin, to produce colorimetric or lateral flow sensors (V. R. Cherkasov, et al., 2020, ACS Nano, 14, 1792-1803).

Regarding miRNA detection, still new systems are required which should retain high sensitivity, given the low concentrations of miRNAs in biological samples.

Another critical challenge is the need to develop systems suitable for multiplexing, as changes in the expression of a single miRNA are generally insufficient to draw valid conclusions in terms of diagnosis. Thus, it is necessary to develop systems able to detect and quantify several miRNAs at the same time, to obtain a valid diagnostic profile (A. S. Kibel, 2009, Urol. Oncol. Semin. Orig. Investig., 27*).* Examples of AuNP-based sensors reported in the literature, however, generally address the detection of a single miRNA to validate the proposed system. Nevertheless, the signature of several diseases involves the dysregulation of more than one miRNA at a time, and for that reason, multiplexing systems are desired to ease diagnosis.

Generally, AuNP-based microarray strategies preserve the advantages of classical microarrays, which include rapid, parallel and high-throughput analyses. Microarrays are a multiplex technique, i.e., they allow the detection of several miRNAs in a single experiment. The use of microarrays is generally less expensive than other profiling techniques, allowing to evaluate the expression of hundreds of miRNAs using only small amounts of total RNA (Dong H, et al., 2013, Chem Rev., 113:6207-33). However, there are some limitations in the standard microarrays, such as low sensitivity, specificity and a restricted linear range that hampers absolute quantification (Flowers E, et al., 2013, Biol Res Nurs., 15:167-78).

Glass slide-based strategies, in particular microarray-based platforms, are the only ones demonstrating high-throughput and multiplexing (Roy S, Soh JH, Ying JY., 2016, Biosens Bioelectron, 75:238-46), but significant work is yet to be done towards the development of other types of sensors with multiplexing abilities.

In summary, it is necessary to establish new tools and methods that are sensitive, simple, cost-effective and suitable for the multiplexing detection of miRNAs involved in diseases such as cancer.

### DESCRIPTION OF THE INVENTION

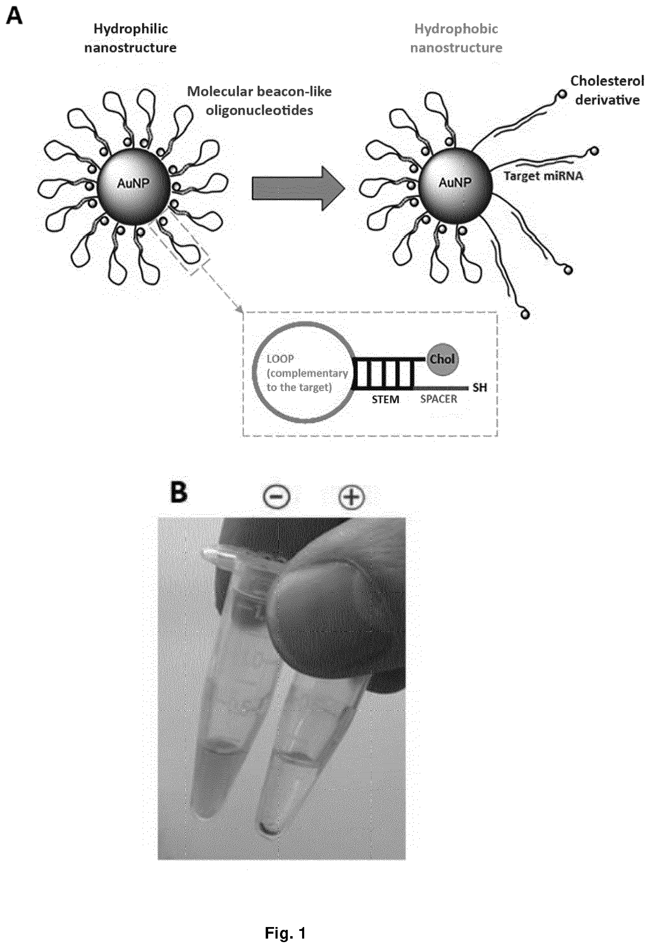

In this invention, metal nanoparticles and an *in vitro* method for the simultaneous or multiplex detection of disease-related nucleic acids, preferably miRNAs, more preferably cancer-related miRNAs, in biological samples are provided. The method is based on the use of a multiplexed colorimetric sensor that comprises metal nanoparticles (NPs), preferably gold NPs (AuNPs), functionalized with different oligonucleotide probes that fold into a hairpin structure and contain a hydrophobic molecule (preferably a cholesterol derivative) at one end, whereas the other end contains a moiety that allows its attachment to the NPs. In the initial arrangement of the system, the hairpin retains the hydrophobic molecule buried inside the nanostructure and the system is well dispersed in aqueous media, presenting the characteristic reddish color due to the plasmon band of NPs. However, in the presence of the complementary target sequence, the hairpin unfolds, exposing the hydrophobic molecule to the water molecules. This leads to the aggregation of the structure, which can be detected with the naked eye because the reddish color disappears (Figure 1). Thus, this multiplex system is based on the modulation of the colloidal stability of the NPs functionalized with different oligonucleotides probes that target different nucleic acids, preferably miRNAs, involved in diseases.

The novelty of the system of the invention resides mainly in its multiplexing capabilities, being able to detect several target nucleic acids, preferably, miRNAs, at a time. Unexpectedly, the multiplexed format improves the system's sensitivity when compared to single target detection, down to picomolar concentrations that can be found in liquid biopsies being thus sufficient to detect nucleic acids, preferably, miRNAs, in said kind of samples (see Examples 4 and 5.2 below). In fact, it shows excellent performance in complex biological samples, as the human serum, allowing for direct and visual determination in liquid biopsies from patients, for example cancer patients. The multiplex method of the invention shows thus a good selectivity, since it is capable of specifically detecting different target miRNAs in complex mixtures, such as biological samples, in which many different molecules and other miRNAs are also present (see Example 5 below).

As mentioned above, in addition to its sensitivity and selectivity, the method of the invention proved to be suitable for multiplexed detection at picomolar concentrations of target sequences. This gain in sensitivity is very relevant, given the low concentrations of miRNAs in biological samples. In this sense, it is worth mentioning that the picomolar range is sufficient to detect these molecules in liquid biopsies. The NPs of the invention are prepared to detect more than one target sequence simultaneously, which increases the sensitivity of NPs targeting only one sequence. The method presented herein can detect at least two, preferably three, miRNA sequences and the limits of detection are improved compared to the use of single oligonucleotide probe nanoparticles.

The multiplex system is proposed to be used, preferably, to simultaneously detect combinations of different miRNAs present and involved in cancers, like uveal melanoma, pancreatic cancer and/or breast cancer. In a particular embodiment of the invention, gold NPs bound to three oligonucleotide probes for the detection of the three miRNAs, 146a, 155 and 191, which are upregulated in cancers like uveal melanoma, pancreatic cancer and breast cancer, are described.

Besides miRNA profiling, the NPs of the invention can be easily tuned for the detection in liquid biopsies of any tumoral DNA or nucleic acid biomarker overexpressed in patient samples, extending the advantages of multiplexed nucleic acid detection to different targets and disease contexts.

Another advantage of the system presented herein is that it is user-friendly and is accessible as a detection device, which will help clinicians to establish a quick and precise diagnosis and prognosis of different diseases with measurable nucleic acid biomarkers in body fluids.

Also, the detection of multiple targets from the same sample decreases the variability due to sample manipulation and/or storage. As mentioned, with the method of the invention, several miRNAs can be detected simultaneously in the same sample, individually or in a mixture, using a single reagent and at concentrations suitable for detection in liquid biopsies. This approach would allow for a faster and more reliable identification of diseased patients, helping in early diagnosis and stratification.

Since the color of the solution changes in the presence of the target sequence, the NPs of the invention can be used directly, with minimal sample treatment, reducing the need for complex purification or amplification procedures. Such fast, reliable, and affordable diagnostic systems are essential to implement early detection strategies in healthcare systems. With the NPs described herein, this medical need is addressed by providing a simple, fast and cost-effective system, which is suitable for use in PoC settings and/or resource-limited facilities. This approach can help greatly to achieve fast and early disease, preferably cancer, detection strategies, through profiling of nucleic acids biomarkers in liquid biopsies.

Summarizing, functionalized NPs with excellent performance in biological samples are proposed in this invention for the multiplex detection of disease-related nucleic acids, preferably cancer-related miRNAs. The system allows visual and direct detection of multiple nucleic acids, preferably miRNAs, with minimal sample treatment. It is sensitive (see Example 2 below), simple, cost-effective and fast, so it can be easily used in point-of-care (PoC), contributing to the fast and early detection of cancer and other diseases.

Therefore, one aspect of the invention refers to a metal nanoparticle (NP, hereinafter "the nanoparticle of the invention" or the "NP of the invention"), preferably a gold nanoparticle (AuNP), bound to at least two oligonucleotides, wherein each oligonucleotide comprises:
(i) an end, preferably the 5' end, bound to the metal nanoparticle,
(ii) followed by a central nucleotide sequence that is complementary to a target disease-related nucleic acid sequence, preferably microRNA (miRNA) sequence, wherein said central nucleotide sequence is different in each oligonucleotide of those bound to the metal nanoparticle and is complementary to a different target disease-related nucleic acid sequence, preferably miRNA sequence, in each oligonucleotide of those bound to the metal nanoparticle,
(iii) two nucleotide sequences that are complementary to each other and that flank the central nucleotide sequence of (ii), and
(iv) a hydrophobic molecule at the end opposite to the end bound to the metal nanoparticle of (i), preferably wherein said hydrophobic molecule is at the 3'end of the oligonucleotide.

The term "nanoparticle" is used to designate colloidal systems of the spherical type, rod type, polyhedron type, etc., or similar shapes, preferably having a size less than 1 micrometer (µm), which are individually found or are found forming organized structures (dimers, trimers, tetrahedrons, etc.), dispersed in a fluid (aqueous solution). In a particular embodiment, the nanoparticles suitable for putting the invention into practice have a size less than 1 µm, generally comprised between 1 and 999 nanometers (nm), typically between 5 and 500 nm, preferably between about 10 and 150 nm. In a particular embodiment, the nanoparticles of the invention typically have a mean particle diameter ranging from 2 to 50 nm, preferably from 5 to 20 nm, more preferably of 12 nm. The mean particle diameter is the maximum mean particle dimension, with the understanding that the particles are not necessarily spherical. The shape of said nanoparticles can widely vary; advantageously, said nanoparticles will adopt any optically efficient shape such as spheres, rods, stars, cubes, polyhedrons or any other variant as well as complex associations of several particles; in a particular embodiment, the shape of the nanoparticles for putting the invention into practice is spherical or substantially spherical. The shape can be suitably evaluated by conventional light or by means of electron microscopy techniques.

The term "metal nanoparticle" refers to a nanoparticle comprising a metal and showing the optical property known as the surface plasmon phenomenon, i.e., a plasmonic metal. This phenomenon consists of the collective vibration of the electrons of the metal surface, producing an absorption band located in the ultraviolet-visible spectrum (typical of the metal and of the size of the nanoparticles) at the wavelength where the resonance condition occurs in said electrons. The surface plasmon of a metal can be determined by means of any spectroscopic technique known in the state of the art, such as surface plasmon resonance (SPR) spectroscopy, whereby the metal atoms are subjected to an electromagnetic beam or surface plasmon resonance fluorescence spectroscopy (SPFS) based on the detection of the variation of the refractive index of the metal atoms when they are subjected to a photon beam. As defined herein, a "plasmonic metal" is a metal characterized by showing the property of optics known as the surface plasmon phenomenon. The variation of the plasmonic response is particularly evident when several nanoparticles are located close to one another, given that this causes the coupling of their respective near fields, generating a new surface plasmon. In a particular embodiment, said metal is selected from the group consisting of gold, silver, copper, aluminum, platinum, iron, cobalt, palladium and combinations thereof. In an even more particular embodiment, the metal forming said nanoparticle is gold.

As it is used herein, the term "oligonucleotide" refers to a nucleic acid preferably of at least 10 nucleotides, preferably of at least 16 nucleotides, preferably of at least 20 nucleotides, preferably of at least 25 nucleotides, and preferably not more than 100 nucleotides, including polyribonucleotides or polydeoxyribonucleotides or combinations thereof. The term "oligonucleotide" also refers to molecules formed by conventional nucleotides bound by conventional phosphodiester bonds as well as variants thereof, including modifications in the purine or pyrimidine residues and modifications in the ribose or deoxyribose residues designed for increasing biological stability of the oligonucleotide or for stabilizing the physical stability of the hairpin shaped structure. Examples of modified nucleotides that can be used in the present invention include, but are not limited to, nucleotides having at position 2' of the sugar a substituent selected from the fluoro, hydroxyl, amino, azido, alkyl, alkoxy, alkoxyalkyl, methyl, ethyl, propyl, butyl group or a functionalized alkyl group such as ethylamino, propylamino and butylamino. Alternatively, the alkoxy group is methoxy, ethoxy, propoxy or a functionalized alkoxy group according to the formula - O(CH2)q-R, where q is 2 to 4 and R is an amino, methoxy or ethoxy group. Suitable alkoxyalkyl groups are methoxyethyl and ethoxyethyl. Oligonucleotides in which different nucleotides contain different modifications at position 2' are also part of the invention. Alternatively or additionally, the oligonucleotides of the invention can contain modified bonds such as phosphodiester-, phosphotriester-, phosphorothioate-, phosphorodithioate-, phosphoroselenoate-, phosphorodiselenoate-, phosphoroanilothioate-, phosphoramidate-, methylphosphonate-, boranephosphonate-type bonds as well as combinations thereof, or they are peptide nucleic acids (PNAs), in which the different nucleotides are bound by amide bonds. The chemical synthesis of nucleic acids is a well-known technique and it can be performed, for example, by means of using an automatic DNA synthesizer (such as commercially available Bioautomation synthesizers, for example). Said oligonucleotide can be synthesized by methods known in the art as the solid-phase phosphoramidite method, the triester method or the phosphorothioate method. For example, said oligonucleotide can be synthesized on a solid support using a MerMade (Bioautomation) synthesizer. Other conventional solid supports for oligonucleotide synthesis that are well known by the person skilled in the art include, but are not limited to, inorganic substances such as silica, porous glass, aluminosilicates, borosilicates, metal oxides, etc. Alternatively, said solid support can be an organic substance, such as a cross-linked polymer, such as polyamide, polyether, polystyrene, etc. The preferred solid support for use in the synthesis of the oligonucleotide forming the nanoparticle according to the present invention is controlled porous glass (CPG).

The two nucleotide sequences indicated in (iii) are also referred to in the present invention as "stem sequences". These two stem sequences indicated in (iii) can hybridize and give rise to a hairpin-type structure in the oligonucleotide.

As it is used herein, the term "complementary" refers to sequences having a degree of complementarity that is sufficient for specifically hybridizing. Absolute complementarity between the sequences described in this invention is not required, although it is preferred. In a preferred embodiment, said degree of complementarity is at least 10%, at least 20%, at least 30%, at least 40%, at least 50%, at least 60%, at least 70%, at least 80%, at least 90%, at least 95%, at least 99%, even more preferably said degree of complementarity is 100%. The degree of complementarity between two sequences is determined by means of comparing the order of the nitrogen bases forming the two sequences without taking into account structural differences between said sequences, provided that said structural differences do not prevent the formation of hydrogen bridges between the complementary bases. The degree of complementarity can also be determined in terms of the number of unpaired bases present between the sequences. As the person skilled in the art will understand, the hybridization capacity will depend on the degree of complementarity between the sequences and on the length of the sequences.

In a preferred embodiment, the two nucleotide sequences indicated in (iii) are of equal length, i.e., they comprise the same number of nucleotides. In another preferred embodiment, said two nucleotide sequences indicated in (iii) are of different lengths. Each of the two nucleotide sequences indicated in (iii) typically comprises 2, 3, 4, 5, 6, 7, 8, 9, 10, 11, 12, 13, 14, 15, 20, 25, 30, 40 or 50 nucleotides. More preferably, each of the two nucleotide sequences indicated in (iii) comprises, even more preferably consists of, 5 nucleotides.

In an even more preferred embodiment, the two nucleotide sequences indicated in (iii) comprise or consist of, respectively, the sequences CGGCC and GGCCG.

Hybridization between the two nucleotide sequences indicated in (iii) gives rise to the formation of a hairpin-type structure in the oligonucleotides bound to the NP. As it is used herein, a "hairpin-type structure" or "hairpin loop" refers to a secondary structure formed by an oligonucleotide comprising two stem regions, wherein the hybridization of said stem regions gives rise to a sequence of a double-stranded oligonucleotide.

The oligonucleotides forming the nanoparticle of the invention comprise a central nucleotide sequence (ii) flanked by said two stem nucleotide sequences indicated in (iii). In a preferred embodiment, said central nucleotide sequence (ii) comprises 5, 6, 7, 8, 9, 10, 11, 12, 13, 14, 15, 20, 21, 22, 23, 25, 30, 40, 50, 60, 70, 80, 90, 95 or 100 nucleotides. Said central nucleotide sequence (ii) comprises a nucleotide sequence complementary to a target disease-related nucleic acid sequence, preferably a disease-related miRNA sequence. In a preferred embodiment, said target disease-related miRNA sequence is miRNA-146a (SEQ ID NO: 4), miRNA-155 (SEQ ID NO: 5) and miRNA-191 (SEQ ID NO: 6), or any combination thereof.

In another preferred embodiment of the metal nanoparticle of the invention, the disease is cancer, preferably uveal melanoma, pancreatic cancer and/or breast cancer.

In another preferred embodiment of the metal nanoparticle of the invention, the central nucleotide sequence of (ii) comprises, more preferably consists of, SEQ ID NO: 1 (AACCCATGGAATTCAGTTCTCA, probe specific for miRNA-146a), SEQ ID NO: 2 (ACCCCTATCACGATTAGCATTAA, probe specific for miRNA-155), SEQ ID NO: 3 (AGCTGCTTTTGGGATTCCGTT, probe specific for miRNA-191), or any combination thereof.

More preferably, the nanoparticle of the invention is bound to three oligonucleotides and one of the three oligonucleotides comprises a central nucleotide sequence comprising or consisting of the SEQ ID NO: 1, other oligonucleotide comprises a central nucleotide sequence comprising or consisting of the SEQ ID NO: 2 and the other oligonucleotide comprises a central nucleotide sequence comprising or consisting of the SEQ ID NO: 3.

In a particular embodiment, the oligonucleotide sequence of the invention is SEQ ID NO: 22 (TTTTTCGGCCAACCCATGGAATTCAGTTCTCAGGCCG), SEQ ID NO: 23 (TTTTTCGGCCACCCCTATCACGATTAGCATTAAGGCCG) or SEQ ID NO: 24 (TTTTTGGCCCAGCTGCTTTTGGGATTCCGTTGGGCC).

The oligonucleotides bound to the metal nanoparticle of the invention have modifications at their 3' and 5' ends. At one of its ends, preferably the 5' end, the oligonucleotide is modified with a suitable group for the binding of the oligonucleotide to the metal nanoparticle. As it is used herein, the term "suitable group for the binding of the oligonucleotide to the metal nanoparticle" refers to a group capable of reacting with the metal atoms of the nanoparticle, allowing the binding between the oligonucleotide and the nanoparticle. In a particular embodiment, said suitable group for the binding of the oligonucleotide to the metal nanoparticle is selected from a group containing a sulfur atom and a polyadenine sequence.

In a particular embodiment, said oligonucleotides bound to the nanoparticle of the invention are modified with a group containing a sulfur atom at one end, preferably the 5' end, and with a hydrophobic molecule at the opposite end, preferably at the 3'end.

The term "group containing a sulfur atom" refers to a set of atoms containing sulfur, which are bound to a carbon chain by means of a covalent bond. The group containing sulfur is preferably associated with the oligonucleotide in the same way that the different nucleotides are associated with one another, i.e., through oxygen at position 3' or at position 5' of the sugar (ribose or deoxyribose) ring. In a particular embodiment, the group containing the sulfur atom is bound to the 5' end of the oligonucleotide. In that case, the group containing sulfur is bound to the oxygen at position 3' of the ribose or deoxyribose ring. Methods for obtaining oligonucleotides modified with groups containing sulfur are known in the state of the art.

In a particular embodiment, the binding between the oligonucleotides and the nanoparticle in the metal nanoparticle of the invention takes place through sulfur-metal bond formed between the metal atoms located on the surface of the nanoparticle and the sulfur group, which is bound to the oligonucleotides. In a preferred embodiment, the sulfur-metal bond is formed by a reaction between the metal atoms of the nanoparticle and a sulfur atom forming part of a functional group containing sulfur. In a preferred embodiment, the sulfur-metal bond is a covalent bond. The terms "covalent", "covalent binding", "covalently bound" must be understood as the formation of a bond between two atoms or groups of atoms by sharing electron pairs. In a particular embodiment, the covalent sulfur-metal bond is formed by means of a reaction between a sulfur atom of a functional group containing sulfur and a metal atom of the nanoparticle. The term "functional group containing sulfur" refers to a set of atoms containing sulfur which are bound to a carbon chain by means of a covalent bond which confer reactivity to the molecule containing them. In a particular embodiment, said functional group containing sulfur is selected from a thiol group, a dithiolane group and an isocyanide group. In another preferred embodiment, the end bound to the metal nanoparticle of (i) comprises a thiol moiety or thiol group.

As it is used herein, "thiol moiety" refers to a molecule formed by a sulfur atom and a hydrogen atom (SH). As it is used herein, an "isocyanide group" is an organic compound with the functional group R-N=C. As it is used herein, "a dithiolane group" refers to a heterocyclic compound derived from cyclopentane containing sulfur formed by means of substituting two methyl groups (CH₂) with two thioether groups.

The substituted methyl groups can be groups located at contiguous positions or at alternating positions in the heterocyclic ring. If two thioether groups substitute two methyl groups located at contiguous positions, then 1,2-dithiolane is obtained. If two thioether groups substitute two methyl groups occupying alternating positions in the heterocyclic molecule, then 1,3-dithiolane is obtained.

In another preferred embodiment, the oligonucleotides bound to the NP of the invention further comprise a spacer sequence between one of the two complementary stem nucleotide sequences of (iii) and the end bound to the metal nanoparticle of (i).

The spacer sequence is preferably located at the 5' end of the oligonucleotide. As it is used herein, "spacer sequence" refers to a nucleotide sequence with which binding with a molecule of interest is carried out, particularly with the group containing the sulfur atom. In a preferred embodiment, the spacer sequence is formed by at least 2 nucleotides, by at least 3 nucleotides, by at least 4 nucleotides, by at least 5 nucleotides, by at least 6 nucleotides, by at least 7 nucleotides, by at least 8 nucleotides, by at least 9 nucleotides, by at least 10 nucleotides or more. In a particular embodiment of the invention, said spacer sequence is a polythymine sequence, more preferably said spacer sequence comprises or consists of 5 thymines (TTTTT). Thus, preferably, the binding between the oligonucleotides and the group containing a sulfur atom is performed through a binding sequence (spacer sequence) formed by 5 thymines.

Polythymine sequences are capable of self-assembling by means of adsorption on the surface of metal particles. The polythymine sequence is preferably associated with the oligonucleotide in the same way that the different nucleotides are associated with one another, i.e., through the oxygen at position 3' or at position 5' of the sugar (ribose or deoxyribose) ring. The binding between said polythymine sequence and the oligonucleotide forming the nanoparticle of the invention takes place through a phosphodiester bond. Methods for obtaining oligonucleotides modified with a polythymine sequence are known in the state of the art. In a particular embodiment, the binding between the polythymine sequence and the nanoparticle is produced by adsorption of said polythymine sequence on the metal atoms of the nanoparticle. As it is used herein, the term "adsorption" refers to the process whereby atoms, ions or molecules are trapped or retained on the surface of a material.

The oligonucleotides bound to the nanoparticle of the invention are modified with a hydrophobic molecule at the end opposite to the end bound to the metal nanoparticle that is modified with a group containing a sulfur atom. Thus, the oligonucleotides bound to the NP of the invention comprise, in addition to the nucleotides forming it, at least one hydrophobic molecule bound, preferably through one of the hydroxyl groups of the sugar molecules forming the nucleotides. As it is used herein, the term "hydrophobic molecule" refers to a molecule that is more soluble in organic solvents (for example, long-chain alcohols, esters, ethers, ketones and hydrocarbons) than in aqueous solutions. Basically, hydrophobicity occurs when the molecule referred to as hydrophobic is not capable of interacting with water molecules through ion-dipole interactions or by means of hydrogen bridges. Hydrophobicity of the "hydrophobic molecule" can be defined by the concentration ratio (partition coefficient) of the molecule distributed between an aqueous phase and an organic phase. Specifically, hydrophobicity can be defined by the partition coefficient log value (partition coefficient P (logP)) between the aqueous and organic phases (for definitions of the terms partition coefficient and partition ratio, refer to "Handbook of Chemistry, 5th ed., Basic II" [The Chemical Society of Japan ed., MARUZEN Co., Ltd., p. 168-177]). In the present invention, "logP" refers to a 20 theoretical distribution coefficient calculated using a logP calculation program (for example, ACD/LogP, (version 9.0)), available from Advanced Chemistry Development, Inc., and an algorithm described in the ACD/LogP manual (2005) included therein. In the present invention, a "hydrophobic molecule" is defined as an organic molecule having a logP of 2 or more, preferably a logP of 7 or more, at pH 7. Specifically, the compound having a logP of 2 or more (i.e., the concentration ratio of the compound between the aqueous and organic phases is 1:100 or more) can be interpreted as having the property of forming aggregates through hydrophobic interactions with other molecules when they are distributed in a neutral aqueous solution.

In another preferred embodiment of the NP of the invention, the hydrophobic molecule of (iv) is a lipid, preferably a cholesterol derivative. As it is used herein, "lipid" refers to organic molecules that have a long-chain fatty acid or a hydrocarbon chain and are slightly soluble in water and readily soluble in organic solvents. In a more particular embodiment, said lipid is a steroid lipid. As it is used herein, "steroid lipid" refers to lipids derived from the nucleus of the hydrocarbon sterane (or cyclopentaneperhydrophenanthrene), being made up of four rings fused with functional hydroxyl and carbonyl groups. In a more particular embodiment, said steroid lipid is cholesterol. As it is used herein, "cholesterol" refers to a sterane molecule having two methyl radicals at positions C10 and C13, a branched aliphatic chain containing eight carbons at position C17, a hydroxyl group at position C3 and an unsaturation between the C5 and C6 carbons; where C refers to a carbon atom, and wherein the number represented next to said C refers to the specific position said carbon atom occupies in the molecule.

Methods for binding a hydrophobic molecule to an end of an oligonucleotide are well known in the state of the art. The incorporation of the hydrophobic group into the oligonucleotide is preferably carried out by means of using precursors of said groups activated with a phosphoramidite group, which allows the incorporation of the hydrophobic group by reacting the phosphoramidite group with a hydroxyl group in the sugar molecule of the oligonucleotide. In a preferred embodiment, the incorporation of the hydrophobic group is carried out by means of using the precursor cholesteryl-3-carboxamidohexyl-[(2-cyanoethyl)-(N,N-diisopropyl)]-phosphoramidite.

The nanoparticle of the invention has amphipathic characteristics, i.e., it has a water-soluble, hydrophilic region in its structure formed by the oligonucleotides forming part of said nanoparticle, and a hydrophobic region formed by the hydrophobic molecule bound to an end of the oligonucleotides. The amphipathicity of the molecule is variable depending on the folding state of the hairpin-shaped region. Thus, if the region with the hairpin structure is folded, the nanoparticle is more hydrophilic, given that the loop of said region, which is hydrophilic, is exposed to the exterior. If the region with the hairpin structure is unfolded, the nanoparticle is more hydrophobic given that the hydrophobic group is exposed to the exterior of the nanoparticle.

NPs of the invention can also be easily adapted for their use in a multiplexed lateral flow format, though the modification of each probe oligonucleotide with a different reporter molecule (e.g., biotin, digoxigenin, fluorescein) and placement of different biorecognition molecules (e.g., streptavidin, antibodies, aptamers) in separate test lines of a lateral flow sensor. With this, individual identification of each nucleic acid biomarker could be possible, along with individual quantification, due to the use of independent reporter and biorecognition molecule pairs and the spatial separation of the detection sites, while retaining the advantages of visual detection, cost-effectiveness, simplicity and detection speed. Thus, in another preferred embodiment of the invention, each of the oligonucleotides bound to the NP of the invention further comprise a different reporter molecule (e.g., biotin, digoxigenin, fluorescein) and/or different biorecognition molecules (e.g., streptavidin, antibodies, aptamers).

Another aspect of the invention refers to an *in vitro* multiplex method, hereinafter "the method of the invention", for the simultaneous detection of more than one disease-related nucleic acid, preferably disease-related miRNA, more preferably cancer-related miRNA, in an isolated biological sample, wherein said method comprises:
a. contacting at least one, preferably more than one, metal nanoparticle of the invention with the isolated biological sample to be tested, and
b. visualizing the aggregation of the metal nanoparticle,
wherein the visualization of aggregation in (b) indicates the presence of the disease-related nucleic acids, preferably disease-related miRNAs, in the sample and therefore the presence of the disease in the subject from whom the sample has been isolated.

Said first step (a) must take place under conditions that allow hybridizing the central nucleotide sequence of the oligonucleotides bound to the NP of the invention with the target nucleotide sequences, preferably target miRNAs, present in said sample.

As it is used herein, the term "hybridization" refers to the formation of a duplex-type structure by two nucleic acids due to complementary base pairing. Hybridization can occur between completely complementary nucleic acid chains or between "substantially complementary" nucleic acid chains containing small unpaired regions. The conditions with which completely complementary nucleic acid chains hybridize are referred to as "strict hybridization conditions" or "sequence-specific hybridization conditions" or "stringent conditions". Nevertheless, substantially complementary stable double strands of nucleic acids can be obtained under less strict or less stringent hybridization conditions, in which case, the tolerated degree of mismatch can be adjusted by means of suitable adjustment of the hybridization conditions. The person skilled in the art can empirically determine the stability of a duplex taking a number of variables into account, such as probe base pair length and concentration, ionic strength and mismatched base pair incidence, following the guidelines of the state of the art (see, for example, Sambrook et al., Molecular 5 Cloning: A Laboratory Manual, Cold Spring Harbor Laboratory, Cold Spring Harbor, N.Y. (1989); and Wetmur, Critical Reviews in Biochem. and Mol. Biol. 26 (3/4):227-259 (1991)). In the context of the present invention, "specific hybridization" is understood as the binding, hybridizing or duplex-forming capacity of the central nucleotide sequence of the oligonucleotides bound to the nanoparticle of the invention with the target sequences to be detected such that said target nucleic acids to be detected can be identified or distinguished from other components of a mixture (for example, cell extracts, genomic DNA, etc.).

In preferred embodiments, specific hybridization is carried out under highly restrictive conditions or under moderately restrictive conditions. Suitable conditions for said hybridization to occur are illustratively shown in Examples of the application. For example, hybridization between the target nucleic acid molecule and the central nucleotide sequence of the oligonucleotides bound the nanoparticle of the invention takes place when about 50-500 µL of nanoparticles at an approximate concentration of 7-15 nM are contacted in about 100-1000 µL of water, or any suitable saline buffer (volume ratio of nanoparticles to buffer of 1:2), with about 5-50 µL of the sample containing the nucleic acid of interest at a concentration of between 0.5 and 1000 nM.

In a preferred embodiment, the central nucleotide sequence of the oligonucleotides bound the nanoparticle of the invention is 100% complementary to the corresponding region of the target nucleic acid, preferably target miRNA, to be detected. In another preferred embodiment, the central nucleotide sequence of the oligonucleotides bound to the nanoparticle of the invention is at least 50%, at least 60%, at least 70%, at least 80%, at least 90%, at least 95%, at least 99% complementary to the corresponding region of the target nucleic acid, preferably target miRNA, to be detected.

As a result of said hybridization between the central nucleotide sequence of the oligonucleotides bound the nanoparticle of the invention and the target sequence, a conformational change occurs in said oligonucleotides because the hybrid that is formed is thermodynamically more stable than the nanoparticle wherein said central nucleotide sequence does not hybridize with said target sequence. Thermodynamic stability can be determined by means of parameters known by the person skilled in the art, such as Gibbs free energy. Said conformational change refers to the fact that the oligonucleotides bound to the nanoparticle of the invention switch from "conformation I" to "conformation II". As it is used herein, the term "conformation I of the oligonucleotides bound to the nanoparticle of the invention" refers to a conformation wherein the oligonucleotides have a hairpin-type structure due to hybridization between the two nucleotide sequences indicated in (iii) that are complementary to each other and that flank the central nucleotide sequence of (ii). In this conformation, the hydrophobic molecule bound to one end of the oligonucleotide is not exposed to the solvent. Said conformation I is the native conformation of the oligonucleotides bound to the nanoparticle of the invention. As it is used herein, the term "conformation II of the oligonucleotides bound to the nanoparticle of the invention" refers to the conformation adopted by said oligonucleotides when the central nucleotide sequence of the oligonucleotides bound the nanoparticle of the invention hybridizes with its target sequence. As a result of said hybridization, the Watson-Crick bonds formed between the complementary nucleotides of two nucleotide sequences indicated in (iii) break. In this conformation II, the hydrophobic molecule bound to the oligonucleotide bound to the nanoparticle of the invention is exposed to the solvent. The term "exposed to the solvent" refers to the fact that said group contacts with the solvent. As discussed above, as a result, a conformational change occurs in said oligonucleotides bound to the nanoparticle of the invention and said nanoparticle switches to a conformation wherein the hydrophobic molecule bound to the oligonucleotide is exposed to the solvent. In said conformation, the nanoparticles tend to associate with one another (aggregate) due to the hydrophobic interactions between them, which will be generated to minimize interaction between the hydrophobic molecule and the aqueous solution in which the present method is carried out. As it is used herein, the term "aggregation" refers to the process whereby two or more individual nanoparticles aggregate as a result of the interaction between them. As a result of said aggregation, aggregates formed by dimers, trimers, and aggregates of a higher order will be generated.

As it is used herein, the term "sample" refers to any material containing nucleic acid, for example, DNA or RNA, preferably RNA, more preferably miRNAs, which is obtained from a biological source or artificially synthesized. As it is used herein, "biological sample" refers to a tissue-, cellular or biological fluid-type material. If the material in which the presence of a certain nucleic acid, preferably miRNA, is to be determined according to the present method is a tissue or a cell, prior extraction of the nucleic acid from the sample is preferably performed using any technique suitable for that purpose. The biological sample can be treated to physically or mechanically break down the tissue or cell structure, releasing the intracellular components into an aqueous or organic solution to prepare the DNA or RNA extracts. In a particular embodiment of the invention, said biological sample is a cell lysate. In another particular embodiment, said biological sample is a biological fluid.

In another preferred embodiment of the method of the invention, the biological sample is a liquid biopsy, preferably the biological sample is selected from the list consisting of: plasma, serum, urine, saliva, seminal fluid, ascites fluid, amniotic pleural effusions, or cerebrospinal fluid, more preferably serum.

The biological sample may be obtained from any human or non-human mammal. Preferably, the biological sample referred to in the present invention is from a human being.

In another preferred embodiment of the method of the invention, the biological sample has been pre-treated before step (a) of the method with Proteinase K followed by heating, in order to minimize possible unspecific interactions with the large plasma proteins. Preferably, the Proteinase K treatment is performed with 0.1-0.5 mg/mL, preferably 0.2 mg/mL, of Proteinase K under incubation conditions of 50-58ºC, preferably 55ºC, during 10-16h, preferably 12h, followed by heating at 90-98ºC, preferably 95ºC, for 6-15 min, preferably 10 min.

Step (b) of the method of the invention, which comprises visualizing the aggregation of the metal nanoparticle, can be done with the naked eye due to changes in the intensity and saturation in the color of the solution. However, in another preferred embodiment of the method of the invention, step (b) is performed by measuring the absorbance of the mixture remaining after step (a). More preferably, the absorbance is measured by UV-visible spectroscopy. Said technique is based on determining the surface plasmon resonance in a suitable wavelength depending on the metal forming the nanoparticles. Thus, in the event that the nanoparticles are gold nanoparticles, the determination of surface plasmon resonance is carried out at a wavelength of between 450-600 nm, preferably about 530 nm.

The absorbance measurement can be carried out after a time interval that is considered suitable. For example, absorbance can be determined after a period of time that is optimal for hybridization to occur between the central nucleotide sequence of (ii) of the oligonucleotides and the target nucleic acids to be determined in the sample and for nanoparticle aggregates to be formed. In a preferred embodiment, the absorbance measurement is determined after a sample-nanoparticle incubation period of at least 2h, preferably at least 4h, more preferably at least 8h. In another preferred embodiment, the absorbance measurement is determined after a sample-nanoparticle incubation period of about 16 hours or 24 hours.

In another preferred embodiment, the method of the invention comprises an additional step (c) in which the NPs of the invention are incubated with a control sample that do not contain the nucleic acid/s, preferably the miRNA/s, to be detected, and the aggregation of these metal nanoparticles are visualized and compared to the aggregation visualized in step (b) for the sample to be tested.

Optionally, the method of the invention may comprise other additional steps. For example, an RNA extraction step can be performed prior to step (a) of the method. This step may be carried out by any of the methods known by the person skilled in the art, including, without being limited to, Trizol^{®}, guanidinium salts, phenol, chloroform, etc. There are also commercial kits that allow extracting RNA from a sample, such as the Qiagen^{®} RNA extraction kit, for example. As the person skilled in the art knows, maximum precautions must be taken when working with RNA to avoid contaminations with RNases and RNA degradation.

Another optional step is an amplification (enrichment) before carrying out the first step of the present method of the invention. Methods for amplifying nucleic acid molecules are known in the art, and include, but are not limited to, polymerase chain reaction (PCR), RT-PCT, ligase chain reaction (LCR), Q-replicase amplification, degenerate oligonucleotide primed-polymerase chain reaction (DOP-PCR), T7/primase-dependent amplification, self-sustained sequence replication (3SR), and isothermal amplification. If this step of amplification is carried out, purified products are not necessary to carry out the first step of the present method of the invention. In a still more particular embodiment, said amplification is carried out by means of isothermal amplification. Briefly, the isothermal amplification methods are based on using a constant temperature rather than cycling through denaturation and annealing extension steps. Known isothermal methods for amplification of nucleic acids include but are not limited to helicase-dependent amplification (HAD), thermostable-HAD, strand displacement amplification (SDA), multiple displacement amplification (MDA), rolling circle amplification, single primer isothermal amplification (SPIA), transcription-mediated amplification (TMA), Nucleic Acid Sequence Based Amplification (NASBA), Loop-mediated isothermal amplification (LAMP) and amplification reaction using nicking enzymes.

In another preferred embodiment of the method of the invention, more than one disease-related miRNA is detected. miRNAs are small non-coding sequences that contains about 20 nucleotides found in plants, animal and some viruses which functions in RNA silencing and post-transcriptional regulation of gene expression.

In a preferred embodiment of the method of the invention, the target disease-related miRNA sequence is miRNA-146a (SEQ ID NO: 4), miRNA-155 (SEQ ID NO: 5) and miRNA-191 (SEQ ID NO: 6), or any combination thereof, even more preferably the target disease-related miRNA sequences are miRNA-146a (SEQ ID NO: 4), miRNA-155 (SEQ ID NO: 5) and miRNA-191 (SEQ ID NO: 6).

In another preferred embodiment of the method of the invention, the disease is cancer, preferably uveal melanoma, pancreatic cancer and/or breast cancer.

In another preferred embodiment of the method of the invention, the central nucleotide sequence of (ii) of the oligonucleotide bound to the NP used is SEQ ID NO: 1 (probe specific for miRNA-146a), SEQ ID NO: 2 (probe specific for miRNA-155), SEQ ID NO: 3 (probe specific for miRNA-191), or any combination thereof. More preferably, the NP of the invention used in the method of the invention is bound to three oligonucleotides and one of the three oligonucleotides comprises a central nucleotide sequence comprising or consisting of the SEQ ID NO: 1, other oligonucleotide comprises a central nucleotide sequence comprising or consisting of the SEQ ID NO: 2 and the other oligonucleotide comprises a central nucleotide sequence comprising or consisting of the SEQ ID NO: 3.

In a particular embodiment, the NP of the invention used in the method of the invention is bound to three oligonucleotides and one of the three oligonucleotides comprises the SEQ ID NO: 22, other oligonucleotide comprises the SEQ ID NO: 23 and the other oligonucleotide comprises the SEQ ID NO: 24.

Preferably, the NPs and the method of the invention have a detection limit of at least 10 nM (1.6 pmoles), more preferably at least 5 nM (0.8 pmoles), even more preferably at least 1 nM (160 fmoles), particularly 0.5 nM, of target molecules.

In another preferred embodiment, the method of the invention is for the diagnosis and/or prognosis of cancer, or for evaluating the response to an anti-cancer treatment in a patient, preferably wherein the cancer is uveal melanoma, pancreatic cancer and/or breast cancer.

Another aspect of the invention refers to the use of the NP of the invention for the simultaneous or multiplex *in vitro* detection of more than one disease-related nucleic acid, preferably disease-related miRNA, more preferably cancer-related miRNA, in an isolated biological sample.

Another aspect of the invention refers to a kit, hereinafter "the kit of the invention", that comprises at least one NP of the invention. Another aspect of the invention, refers to the use of this kit for the simultaneous or multiplex *in vitro* detection of more than one disease-related nucleic acid, preferably disease-related miRNA, more preferably cancer-related miRNA, in an isolated biological sample.

In the context of the present invention, "kit" is understood as a product of the different reagents for using the nanoparticles of the present invention, preferably for performing the method of the invention. Nevertheless, if the kits defined in the present invention do not comprise the reagents necessary for putting the methods of the invention into practice, such reagents are commercially available and can be found as part of a kit. Suitable materials for packaging the components of the kit include, without being limited to, glass, plastic (polyethylene, polypropylene, polycarbonate and the like), bottles, vials, paper, sachets and the like. Kits can additionally contain instructions for using the different components in the kit. Said instructions can be in printed format or in an electronic device capable of storing instructions such that they can be read by a person, such as electronic storage media (magnetic discs, tapes and the like), optical means (CD-ROM, DVD, USB) and the like. The media can additionally or alternatively contain Internet addresses where said instructions are provided. In a more particular embodiment, the kit of the invention comprises suitable reagents for carrying out the method of the invention.

### DESCRIPTION OF THE DRAWINGS

**Fig. 1****. Schematic representation of the proposed miRNA sensors and the molecular mechanism involved in the detection process.** (A) Gold NPs (AuNPs) functionalized with molecular beacon-like oligonucleotide probes. In the presence of the target miRNA, the hairpin structure unfolds, exposing the cholesterol moiety and leading to the aggregation of the AuNPs. Inset: structure of the oligonucleotides used in this invention. The oligonucleotides contain a thiol moiety (SH) at the 5'-end and a cholesterol derivative (Chol) at the 3'-end. The central sequence of the oligonucleotide (loop) is complementary to the target miRNAs, and is flanked with additional sequences that recognize each other (stem). Therefore, the oligonucleotide can fold, yielding a stable hairpin structure. A spacer of 5 thymines is also included between the stem sequence and the thiol moiety, used to functionalized the AuNPs. (B) Left - sample with AuNPs dispersed in water; right - aggregated nanoparticles in the presence of the target sequence.

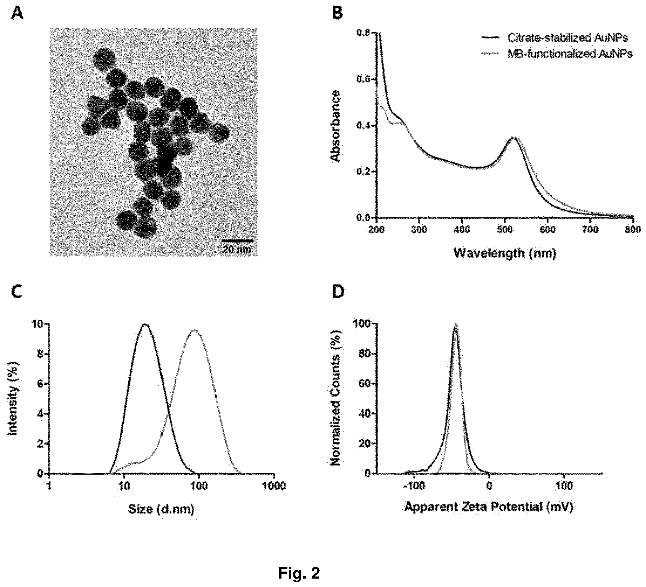

**Fig. 2****. Characterization of the AuNPs used in this invention, both citrate-stabilized and functionalized with the molecular beacon (MB)-like oligonucleotide probe (in this case MB-146a).** A: TEM image of citrate-stabilized AuNPs. B-D: UV-Vis spectra (B), hydrodynamic diameter (C) and Zeta potential (D) of citrate-stabilized (black) and MB-functionalized (grey) AuNPs.

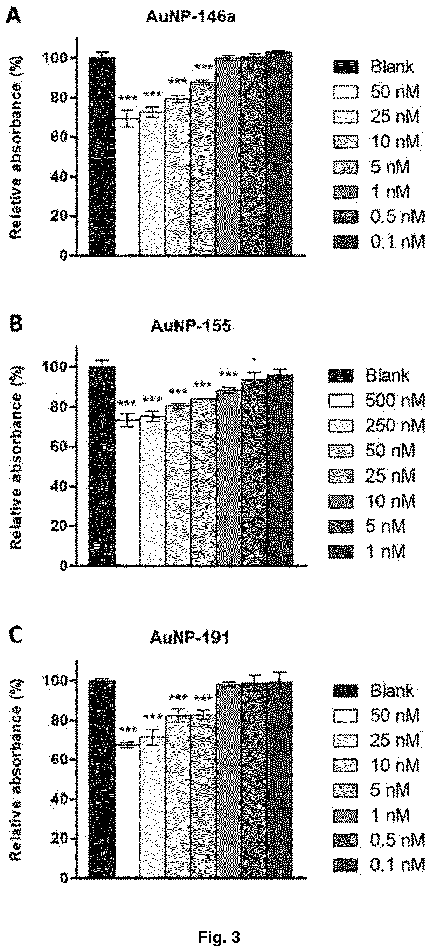

**Fig. 3****. LOD determination for AuNP-146a (A), AuNP-155 (B) and AuNP-191 (C).** For each miRNA sensor, plots of the UV-Vis absorbance maxima at around 530 nm are shown. The RNA sequences of miR-146a, 155 and 191 were added at the indicated concentrations. Presented data were collected after 2h incubation with the target. Results are shown as Mean ± SD (n=3) and relative (in percentage) to the blank value (no target). (.) denotes a significant difference when p < 0.1, compared to the value of the blank samples; (***) when p < 0.001. Statistical analyses were performed by one-way ANOVA with Tukey's post hoc test.

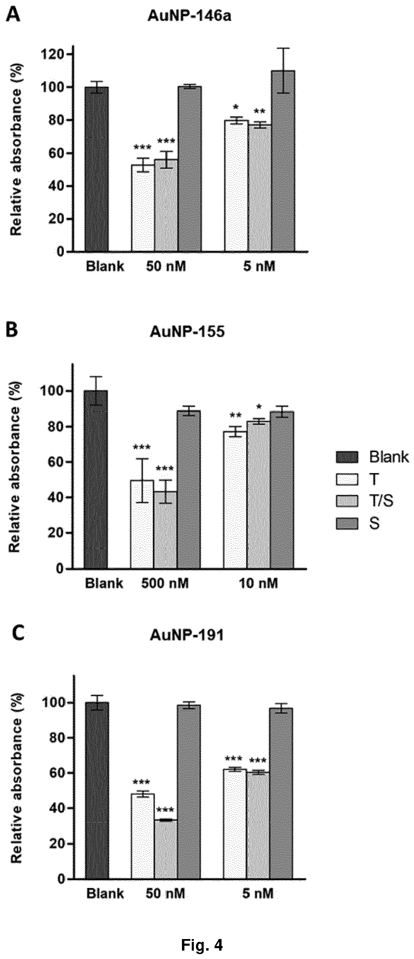

**Fig. 4****. Selectivity studies for AuNP-146a (A), AuNP-155 (B) and AuNP-191 (C).** For each miRNA sensor, plots of the UV-Vis absorbance maxima at around 530 nm are shown. In addition to a non-RNA sample (Blank), for each of the indicated concentrations, three samples were prepared: a sample containing only the target at the indicated concentration (T); a mixture of the target and three scramble sequences (R-1, R-2 and R-3), each one at the indicated concentration (T/S); a sample containing only the three scramble sequences (S). Presented data were collected after 8h incubation with the RNA sequences. Results are shown as Mean ± SD (n=3) and relative (in percentage) to the blank value. (*) denotes a significant difference when p < 0.05, compared to the value of the blank samples; (**) when p < 0.01; (***) when p < 0.001. Statistical analyses were performed by one-way ANOVA with Tukey's post hoc test.

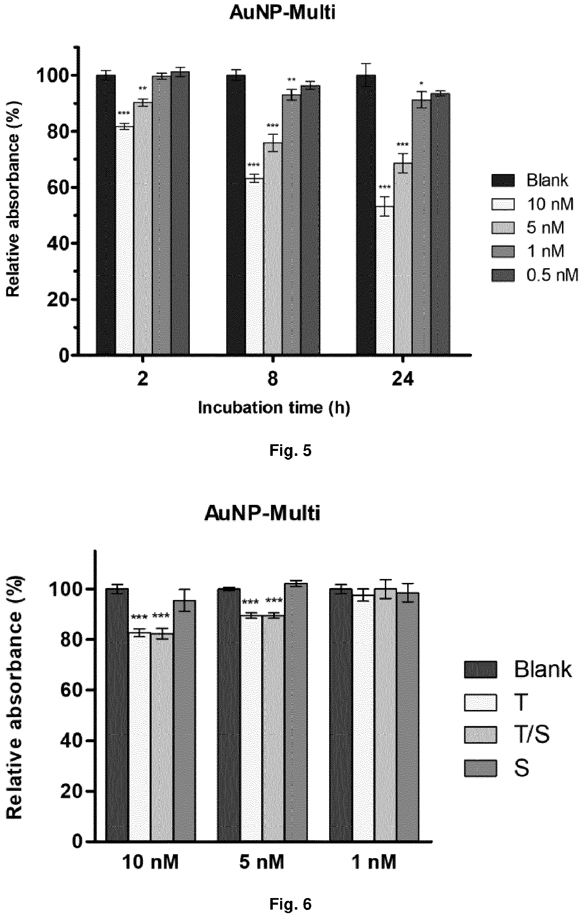

**Fig. 5****. Multiplexed detection of the targets miR-146a, miR-155 and miR-191 using AuNP-Multi.** Plots of the UV-Vis absorbance maxima at around 530 nm are shown. The indicated concentrations reflect the final concentration of targets in each sample. Presented data were collected after 2h, 8h and 24h incubation with the targets. Results are shown as Mean ± SD (n=3) and relative (in percentage) to the blank value (no targets). (*) denotes a significant difference when p < 0.05, compared to the value of the blank samples; (**) when p < 0.01; (***) when p < 0.001. Statistical analyses were performed by one-way ANOVA with Tukey's post hoc test.
**Fig. 6****. Multiplexed detection of the targets miR-146a, miR-155 and miR-191 using AuNP-Multi, in the presence of scramble sequences.** Plots of the UV-Vis absorbance maxima at around 530 nm are shown. In addition to a non-RNA sample (Blank), for each of the indicated concentrations, three samples were prepared: a sample containing the three targets, at the indicated total concentration (T); a mixture of the three targets and the six scramble sequences (R-1 to R-6), each one at the same concentration of each target (T/S); a sample containing only the six scramble sequences (S). Presented data were collected after 2h incubation with the RNA sequences. Results are shown as Mean ± SD (n=3) and relative (in percentage) to the blank value. (***) denotes a significant difference when p < 0.001, compared to the value of the blank samples. Statistical analyses were performed by one-way ANOVA with Tukey's post hoc test.

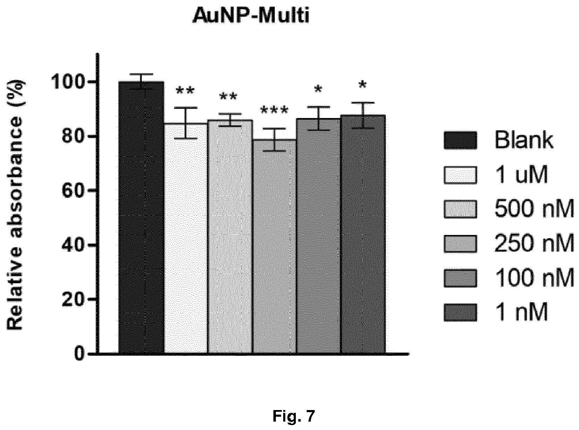

**Fig. 7****. Multiplexed detection of the targets miR-146a, miR-155 and miR-191, added to the reaction in Proteinase K-treated serum.** Plots of the UV-Vis absorbance maxima at around 530 nm are shown. The indicated concentrations reflect the final concentration of targets in each sample. Presented data were collected after 4h incubation with the samples. Results are shown as Mean ± SD (n=3) and relative (in percentage) to the blank value (no targets). (*) denotes a significant difference when p < 0.05, compared to the value of the blank samples; (**) when p < 0.01; (***) when p < 0.001. Statistical analyses were performed by one-way ANOVA with Tukey's post hoc test.

### Examples

*Material and reagents:* The oligonucleotides used in this invention (see Table 1 below) were purchased from Integrated DNA Technologies (IDT, Coralville, IA, USA). Chloroauric acid (HAuCl₄) and sodium chloride (NaCI) were purchased from ThermoFisher Scientific (Waltham, MA, USA). Sodium citrate, phosphate buffered saline (PBS) and human serum from male AB plasma were purchased from Sigma Aldrich (San Luis, MO, USA).

*Preparation of functionalized AuNPs:* Gold nanoparticles (AuNPs) were prepared by the Turkevich method (J. Kimling, et al., 2006, J. Phys. Chem. B, DOI 10.1021/jp061667w.) mixing 34 mg of chloroauric acid (HAuCl₄) with 118 mg of sodium citrate in boiling water. After overnight stirring at room temperature, the AuNP solution was filtered with a 0.3 µm filter with the help of a vacuum pump. Functionalized AuNPs were obtained by the addition of 1-2 pmol of oligonucleotide per µL of 7 nM AuNP solution, in order to achieve maximum surface coverage, with a twice excess. Then NaCl was added gradually to 0.3 M. The obtained AuNPs were incubated at room temperature overnight and then washed by iterative centrifugation using Milli-Q^{®} water (Millipore, Burlington, MA, USA).

*Characterization of functionalized AuNPs:* Gold nanoparticles were visualized by TEM using a 120 KeV JEM1400 Flash (Jeol, Akishima, Tokyo, Japan) at the CBMSO-CSIC institute. Samples were prepared by placing a glow-discharged carbon-shadowed formvar-coated HEX 400-mesh copper grid (Agar Scientific, Stansted, Essex, UK) over one drop of the AuNP solution for 2 min and drying the excess of sample. The shape and size distributions were determined through an automated analysis of randomly selected areas in the TEM images, using Image J software. The mean hydrodynamic diameter and Zeta potential of the AuNPs were measured using a Zetasizer Nano ZS instrument (Malvern Panalytical, Malvern, UK). Absorbance spectra were collected in a Cary 5000 UV-Vis-NIR spectrophotometer (Agilent, Santa Clara, CA, USA).

*Detection of miRNAs in physiological buffer:* For the detection of miRNAs, 50 µL of functionalized AuNPs were treated with 5 µL of target DNA sequences (at different concentrations), 5 µL of NaCl 5 M and PBS, for a constant final volume of 160 µL, in 1.5 mL SuperClear^{®} microcentrifuge tubes (Labcon, Petaluma, CA, USA). For absorbance measurements, 70 µL of each sample were transferred to a UV-Vis absorbance 96-well microplate and the spectra from 400 to 650 nm were collected using a Synergy H4 microplate reader (BioTek, Winooski, VT, USA). Then the absorbance maxima were determined. Experiments were done in triplicates and data were collected at different time points, as well as photographs of the samples.

*Detection of miRNAs in human serum:* For detection in human serum, stock solutions of the mixtures of miRNA sequences were prepared in 150 µL of commercial human serum, at different concentrations. The solutions were treated with 0.2 mg/mL of Proteinase K and incubated at 55 °C for 12h, followed by heating at 95 °C for 10 minutes. Then, 50 µL of functionalized AuNPs were treated with 5 µL of the Proteinase K-treated miRNA stock solutions, 5 µL of NaCl 5 M and PBS, for a constant final volume of 160 µL, in 1.5 mL SuperClear^{®} microcentrifuge tubes (Labcon, Petaluma, CA, USA). Absorbance measurements, data collection and analysis were performed as described for the detection in physiological buffer.

*Statistical analysis:* All experiments were performed in triplicate and the results are presented as mean values ± standard deviation (SD). Statistical analyses were performed using one-away analysis of variance (ANOVA) in order to compare multiple groups. Differences were considered significant at p<0.1. Statistical significance was denoted if the p value was lower than 0.1 by . (p<0.1), 0.05 by * (p<0.05), ** (p<0.01) and *** (p<0.001). The analyses were performed using "R" software program.

### Example 1. Preparation and characterization of gold nanoparticle-based sensors.

For the preparation of the sensors, citrate-stabilized AuNPs of 12 nm were prepared following the Turkevich approach and modified with oligonucleotides that contain a thiol moiety at one end and a cholesterol derivative at the other, folding into a hairpin structure (Figure 1). The sequences employed for the preparation of the nanostructures and the detection assays are summarized in Table 1.

**Table 1. Oligonucleotide sequences employed in this invention.**

| Name^{a)} | Sequences^{b)} |
|---|---|
| Hairpin Oligonucleotide Probes | |
| MB-146a | 5' Thiol- TTTTTCGGCCAACCCATGGAATTCAGTT CTCAGGCCG-Chol 3' (SEQ ID NO: 22) |
| MB-155 | 5' Thiol- TTTTTCGGCCACCCCTATCACGATTAGC ATTAAGGCCG-Chol 3' (SEQ ID NO: 23) |
| MB-191 | 5' Thiol- TTTTTGGCCCAGCTGCTTTTGGGATTCC GTTGGGCC-Chol 3' (SEQ ID NO: 24) |
| RNA Target Sequences | |
| miR-146a | 5' rUrGrArGrArArCrUrGrArArUrUrCrCrArUrGr GrGrUrU 3' (SEQ ID NO: 4) |
| miR-155 | 5' rUrUrArArUrGrCrUrArArUrCrGrUrGrArUrAr GrGrGrGrU 3' (SEQ ID NO: 5) |
| miR-191 | 5’ rCrArArCrGrGrArArUrCrCrCrArArArArGrCr ArGrCrUrG 3’ (SEQ ID NO: 6) |
| RNA Scramble Sequences | |
| R-1 | 5’ rUrGrUrArGrArGrArUrGrCrGrGrUrGrGrUrCr CTT 3’ (SEQ ID NO: 7) |
| R-2 | 5’ rArCrArUrCrUrCrUrArCrGrCrCrArCrCrArGr GTT 3’ (SEQ ID NO: 8) |
| R-3 | 5' rGrUrCrUrCrCrGrArArCrGrUrGrUrCrArCrGr UTT 3' (SEQ ID NO: 9) |
| R-4 | 5' rCrUrArCrGrCrCrArUrCrArGrCrUrCrCrArAr CTT 3' (SEQ ID NO: 10) |
| R-5 | 5’ rArArGrArCrGrGrGrArGrArArGrArGrArArGr GrGrA 3’ (SEQ ID NO: 11) |
| R-6 | 5' rUrUrArUrUrGrCrUrUrArArGrArArUrArCrGr CrGrUrArG 3' (SEQ ID NO: 12) |

| DNA Target Sequences | |
|---|---|
| T-146a | 5' TGAGAACTGAATTCCATGGGTT 3' (SEQ ID NO: 13) |
| T-155 | 5' TTAATGCTAATCGTGATAGGGGT 3' (SEQ ID NO: 14) |
| T-191 | 5' CAACGGAATCCCAAAAGCAGCAGCTG 3' (SEQ ID NO: 15) |
| DNA Scramble Sequences | |
| D-1 | 5' CATCTTCGGCAACCAGATCATCCC 3' (SEQ ID NO: 16) |
| D-2 | 5' TCTCTGACCTTAGGCCCCCTAC 3' (SEQ ID NO: 17) |
| D-3 | 5’ GAGGTTAACAGAGTGACAGATGGTGCA 3’ (SEQ ID NO: 18) |
| D-4 | 5' TGGAATGTAAAGAAGTATGTAT 3' (SEQ ID NO: 19) |
| D-5 | 5' AAGACGGGAGAAGAGAAGGGA 3' (SEQ ID NO: 20) |
| D-6 | 5' ACGATACAGAGAAGATTAGCATGGCCC 3' (SEQ ID NO: 21) |

| | |
|---|---|
| a) Sequence names are composed of letters and numbers. miRNA sequences are designated by "miR" and DNA mimics of the target miRNAs are designated with the letter T ("target"). Oligonucleotide probes are designated with the letters MB ("molecular beacon") and the stem sequences are indicated in bold. The spacer of 5 thymines (T) is also included. The numbers indicate the corresponding miRNA. Scramble sequences are designated with the letter R for RNA sequences and with the letter D for DNA sequences, followed by numbers from 1 to 6. b) Ribonucleotides are indicated with an "r". Sequence modifications: *Thiol* - thiol derivative; *Chol -* cholesterol derivative. | |

The obtained citrate-stabilized AuNPs were imaged by TEM, mostly showing the expected spherical morphology and a mean diameter of 12 nm (Figure 2A). These AuNPs were modified with the thiolated oligonucleotides and the resulting nanostructures were characterized by UV, DLS and Zeta potential (Figure 2B-D). The hydrodynamic diameter of the nanoparticles increased, while the Zeta potential remained unchanged, which was expected after the functionalization, due to the exchange of the citrate molecules with the oligonucleotides, both negatively charged. Regarding the characteristic plasmon band at 520 nm of AuNPs, neither the wavelength nor the intensity of the band changed significantly after the functionalization, highlighting the stability of the nanostructure.

### Example 2. Sensor sensitivity.

The sensing properties of the modified nanoparticles were evaluated. The three sensors (designated AuNP-146a, AuNP-155 and AuNP-191) were assessed in the presence of their corresponding RNA target sequence (miR-146a, miR-155 and miR-191) at different concentrations (Figure 3). The complete evolution, over 24h, was analyzed. After 2 hours, the three systems were able to detect their complementary sequences at least at 10 nM (1.6 pmoles of target molecules), highlighting the versatility of the approach. Remarkably, the sensors for miR-146a and 191 detected the samples at 5 nM (0.8 pmoles), while miR-191 was also detectable at 1 nM (160 fmoles) after 24h.

It was also tested the sensing systems in the presence of DNA strands containing the sequences of the selected miRNAs (T-146a, T-155 and T-191), obtaining sensitivities between 50 and 5 nM.

In summary, the sensors proposed here showed good sensitivity for the direct detection of short RNA and DNA sequences in the nanomolar range, which corresponds to a few pmoles of the nucleic acids.

### Example 3. Sensor selectivity.

Since biological samples constitute a complex mixture, where many different biomolecules and other miRNAs are present, it was next sought to evaluate the selectivity of the proposed sensors in the presence of multiple RNA sequences. For that, three RNA scramble sequences (Table 1) were added to the reaction mixture along with the respective target. In the case of AuNP-146a and AuNP-191 the selectivity was evaluated at 50 and 5 nM of each miRNA, while for AuNP-155 the selectivity was assessed at 500 and 10 nM. The results for the three sensors are shown in Figure 4. The complete evolution of the absorbance during the 24h detection experiment was analyzed. The results revealed that all systems were able to detect with high selectivity their corresponding target sequence (T), at different concentrations, even when mixed with other sequences.

Similar results were obtained when DNA sequences (T-146a, T-155 and T-191) were used instead of RNA, in the presence of three DNA scramble sequences (Table 1), highlighting the system's versatility in detecting different nucleic acids selectively.

### Example 4. Simultaneous detection of three miRNAs - multiplexing.

The results shown above demonstrate that the system developed presents good sensitivity and selectivity, two critical parameters in the design of miRNA sensors, due to their characteristics of low abundance and sequence similarity. However, the signature of several diseases involves the dysregulation of more than one miRNA at a time, and for that reason, multiplexing systems are desired to ease diagnosis. Thus, it was decided to assess the potential of this approach to detect multiple miRNAs. In this case, AuNPs were modified with the three oligonucleotide probes (MB-146a, MB-155 and MB-191, table 1), each one constituting 1/3 of the total MB quantity added (AuNP-Multi). This "hybrid" sensor was used to detect a mixture of the three target miRNAs (miR-146a, miR-155 and miR-191), also each one constituting 1/3 of the total quantity of target sequences. Each one of the target sequences was added at the following concentrations: 3.33, 1.67, 0.33 and 0.17 nM; making up respectively 10, 5, 1 and 0.5 nM of final concentration of targets in the sample. The results are shown in Figure 5. The complete evolution during the 24h experiment was also analyzed.

Similar to the previous individual target detection experiments, UV-Vis spectroscopy monitoring showed a decrease in absorbance maxima with increasing concentration of the target oligonucleotides (Figure 5). The aggregation of the AuNPs was observed after 2h for the 10 and 5 nM samples. Longer incubation made the aggregation more evident, which also allowed the detection of the samples at 1 nM after 8h (Figure 5), improving the limit of detection compared to the individual systems. Similar results were obtained when DNA mimic sequences were employed, detecting samples at 1 nM after 8h and even 0.5 nM after 24h.

Interestingly, taking into account that each individual target is at 1/3 of the total concentration of targets, it is possible to conclude that the sensitivity of AuNP-Multi for each individual miRNA has been significantly improved (three-fold), reaching a LOD of around 330 pM (53 fmoles) after 8h. So, adding to its sensitivity and selectivity, this system proved to be suitable for multiplexed detection at picomolar concentrations, corresponding to fmol levels of target sequences. This gain in sensitivity is very relevant, given the low concentrations of miRNAs in biological samples. In this sense, it is worth mentioning that the picomolar range could be sufficient to detect these molecules in liquid biopsies. Moreover, the sensor could be prepared to detect additional sequences simultaneously, which might increase the sensitivity even further.

The AuNP-Multi system was also tested for the detection of 10, 5, 1 and 0.5 nM concentrations of the individual targets miR-146a, miR-155 and miR-191. The system could detect the three miRNA sequences and the limits of detection were improved, compared to the use of single oligonucleotide probe nanoparticles. In this case, the detection limits of the systems were as follows, 0.5 nM for miR-146a at 24h; 5 nM at 4-8h and 1 nM at 24h for miR-155; 1 nM at 4-8h and 0.5 nM at 24h for miR-191. This improvement in the sensitivity of the AuNP-Multi system, compared to the individual sensors, may be due to the unfolding of oligonucleotide probes (MBs) on many different nanoparticles instead of unfolding many strands in less number of nanoparticles.

The sensitive discrimination of the behavior of different biomarkers at a time, through multiplexing, is desired for miRNA-based diagnosis, since it requires the variation of several miRNAs. Also, the detection of multiple targets from the same sample decreases the variability due to sample manipulation and/or storage. With this method, several miRNAs can be detected simultaneously in the same sample, individually or in a mixture, using a single reagent and at concentrations suitable for detection in liquid biopsies. This approach would allow for a faster and more reliable identification of diseased patients, helping in early diagnosis and stratification.

### Example 5. Multiplexed detection in complex media.

### 5.1. Multiplexed detection in the presence of scramble sequences.

Then, it was examined the feasibility of the multiplexed detection system of the invention (AuNP-Multi) with the presence of six scramble sequences (Table 1). Similarly to selectivity assays, blank samples, samples containing the three targets mixed with the six scramble sequences, only the targets, or only the scramble sequences were prepared. The studies were performed at the concentrations of 10, 5 and 1 nM, so each target was added at the concentrations of 3.33, 1.67 and 0.33 nM. For scramble sequences, in turn, each one was added at the same concentration of the targets, thus making up respectively 20, 10 and 2 nM final concentration of scramble sequences. The results are shown in Figure 6 and the complete evolution of the absorbance over the 8h of the experiment was also assessed.

For the highest concentration tested, 10 nM of targets, slight aggregation could be detected at 2h, but it was clearer from 4h. In the case of the 5 nM concentration, slight aggregation could be observed after 3h and was more evident at 4h. Such aggregations were also selective for the presence of the target sequences in all cases. UV-Vis absorbance measurements confirmed the visual results already at 2h (Figure 6) and other time points. However, at the lowest concentration tested, 1 nM, no aggregation was observed, unlike the previous cases where the scramble sequences were not added (Figure 6).

Detection using the DNA mimic sequences T-146a, T-155 and T-191, along with the DNA scramble sequences (Table 1), produced similar results and even showed selectivity in the detection of 1 nM of target sequences.

In general, this multiplexed detection system (AuNP-Multi) was selective for the presence of the three target miRNA sequences, being able to discriminate them from a mixture of diverse sequences at different concentrations. These results further validate the system for multiplexed detection of miRNAs in complex samples and its possible application in miRNA profiling.

### 5.2. Multiplexed detection in human serum.

To move one step further in the evaluation of the sensor, it was studied the multiplexed detection of the targets miR-146a, miR-155 and miR-191 in human serum. For that, different concentrations of the miRNA sequences (from 1 µM to 1 nM) were pre-diluted in commercial human serum and added, in 5 µL, to the standard sensor reaction mixture. Also, a Proteinase K (PK) treatment, followed by heating, was performed to minimize possible unspecific interactions with the large plasma proteins.

Under these conditions, the system was able to detect the target sequences at different concentrations. The results are shown in Figure 7 and the complete evolution of the absorbance, compared to the detection in water, was also assessed. Detection down to 1 nM (330 pM each miRNA) was achieved at 4h in PK-treated serum, even faster than with the samples in water.

These results show that, in addition to the detection in complex mixtures, the system performs exceptionally well in complex biological environments like human serum. The sensor can recognize specifically several up-regulated miRNAs at a time in biological samples, at the low concentration levels found in liquid biopsies.

Liquid biopsies have received increasing attention in cancer diagnosis, since biomarkers in the patient's blood, like tumor-related nucleic acids (e.g., ctDNAs, miRNAs) or tumor exosomes (containing miRNAs), can be found at early stages. Early diagnosis tools like liquid biopsy analyses can support early decisions for treatment procedures, increasing the chances of successful treatment and survival. Particularly, this statement holds true for tumors like breast cancer, pancreatic cancer and uveal melanoma, where the life expectancy of the patients essentially depends on the timing of the diagnosis and treatment. These examples have focused on the detection of miRNAs present in these cancers, in particular miRNAs 146a, 155 and 191.

In this invention, it is described the development of a sensor based on gold nanoparticles (AuNPs) and oligonucleotides, for the detection of nucleic acids in biological samples. The recognition of the target sequences changes the colloidal stability of the AuNPs, producing color changes that can be easily detected with the naked eye. This system is to be applied to detect miRNAs present in several cancers, like uveal melanoma, pancreatic cancer and breast cancer. The sensor demonstrates good sensitivity and selectivity in the detection of single miRNAs and can also recognize specifically several miRNAs at a time. These multiplexing capabilities allow for improved sensitivity, reaching picomolar levels that can be found in liquid biopsies. The system shows great performance in complex mixtures and biological media, like human serum, being suitable for determination in liquid biopsies with minimal sample treatment. Due to its good performance, simplicity, cost-effectiveness and speed, this system can be easily used in PoC, and can be tuned for the detection of any nucleic acid biomarker in patient samples, contributing to fast and early detection of cancer and other diseases.

## Claims

1. A metal nanoparticle, preferably a gold nanoparticle, bound to at least two oligonucleotides, wherein each oligonucleotide comprises:
(i) an end, preferably the 5' end, bound to the metal nanoparticle,
(ii) a central nucleotide sequence that is complementary to a target disease-related microRNA (miRNA) sequence, wherein said central nucleotide sequence is different in each oligonucleotide of those bound to the metal nanoparticle and is complementary to a different target disease-related miRNA sequence in each oligonucleotide of those bound to the metal nanoparticle,
(iii) two nucleotide sequences that are complementary to each other and that flank the central nucleotide sequence of (ii), and
(iv) a hydrophobic molecule at the end opposite to the end bound to the metal nanoparticle of (i), preferably wherein said hydrophobic molecule is at the 3'end of the oligonucleotide.

2. The metal nanoparticle according to claim 1, wherein the end bound to the metal nanoparticle of (i) comprises a thiol moiety.

3. The metal nanoparticle according to claims 1 or 2, wherein the oligonucleotide further comprises a spacer sequence between one of the complementary nucleotide sequences of (iii) and the end bound to the metal nanoparticle of (i).

4. The metal nanoparticle according to claim 3, wherein said spacer sequence is a polythymine sequence.

5. The metal nanoparticle according to any one of claims 1 to 4, wherein the hydrophobic molecule of (iv) is a lipid, preferably a cholesterol derivative.

6. The metal nanoparticle according to any one of claims 1 to 5, wherein the disease is cancer, preferably uveal melanoma, pancreatic cancer and/or breast cancer.

7. The metal nanoparticle according to any one of claims 1 to 6, wherein the target disease-related miRNAs are miRNA-146a, miRNA-155 and miRNA-191, or any combination thereof.

8. The metal nanoparticle according to any one of claims 1 to 7, wherein the central nucleotide sequence of (ii) is SEQ ID NO: 1, SEQ ID NO: 2, SEQ ID NO: 3, or any combination thereof.

9. The metal nanoparticle according to any one of claims 1 to 8, wherein said nanoparticle is bound to three oligonucleotides and wherein one of the oligonucleotides comprises a central nucleotide sequence comprising the SEQ ID NO: 1, other oligonucleotide comprises a central nucleotide sequence comprising the SEQ ID NO: 2 and the other oligonucleotide comprises a central nucleotide sequence comprising the SEQ ID NO: 3.

10. An *in vitro* multiplex method for the simultaneous detection of more than one disease-related miRNA, preferably cancer-related miRNA, in an isolated biological sample, wherein said method comprises:
a. contacting at least one metal nanoparticle according to any one of claims 1 to 9 with the isolated biological sample to be tested, and
b. visualizing the aggregation of the metal nanoparticle,
wherein the visualization of aggregation in (b) indicates the presence of the disease-related miRNAs in the sample.

11. The method according to claim 10, wherein the biological sample is a liquid biopsy, preferably wherein the biological sample is selected from the list consisting of: plasma, serum, urine, saliva, seminal fluid, ascites fluid, amniotic pleural effusions, or cerebrospinal fluid, more preferably serum.

12. The method according to any of claims 10 or 11, wherein the biological sample is from a human being.

13. The method according to any one of claims 10 to 12, wherein the biological sample has been pre-treated before step (a) with Proteinase K followed by heating.

14. The method according to any one of claims 10 to 13, wherein step (b) is performed by measuring the absorbance.

15. The method according to any one of claims 10 to 14, wherein the cancer is uveal melanoma, pancreatic cancer and/or breast cancer.
